# EUROPEAN PATENT APPLICATION

(11) **EP 4 776 012 A1**
(43) Date of publication of application: **15.07.2026**
(21) Application number: 25151568.0
(22) Date of filing: 13.01.2025
(51) Int. Cl.: G01R 33/54, A61B 5/055, G01R 33/56, G01R 33/24, G01R 33/387, G01R 33/48, G01R 33/561, G01R 33/563

(54) **AUTOMATICALLY OPTIMIZED MR IMAGING**

(71) Applicant: Koninklijke Philips N.V., 5656 AE Eindhoven (NL)
(72) Inventor: BECK, Gabriele, Eindhoven (NL)
(74) Representative: Philips Intellectual Property & Standards

(57) **Abstract**

The invention relates to a method of MR imaging which addresses the interplay of patient-induced artifacts, operational complexity, and the need for precise imaging of clinically relevant areas. The method comprises: acquiring MR imaging data from a patient's anatomy by executing at least one (pre-) scan to generate an image of the anatomy and to extract information including at least one of coil sensitivity distribution, Bu field distribution, Bl field distribution, spatial distribution of flow characteristics, spatial distribution of motion characteristics; identifying an anatomic structure or region of interest within the imaged anatomy; automatically adapting settings of an MR scanner used and/or parameters of an MR imaging sequence or protocol taking the extracted information into account for enhancing image quality or diagnostic utility for the identified anatomic structure or region of interest; and acquiring further MR imaging data from the patient's anatomy using the MR imaging sequence or protocol with the adapted settings and/or parameters to generate one or more optimized images of the identified anatomic structure or region of interest. Moreover, the invention relates to an MR imaging system and to a computer program.

## Description

### FIELD OF THE INVENTION

The present invention relates to the field of magnetic resonance (MR). It finds particular application in conjunction with MR imaging methods and MR scanners for diagnostic purposes, and it will be described with particular reference thereto.

### BACKGROUND OF THE INVENTION

Image-forming MR methods which utilize the interaction between magnetic fields and nuclear spins in order to form two-dimensional or three-dimensional images are widely used nowadays, notably in the field of medical diagnostics, because for the imaging of soft tissue they are superior to other imaging methods in many respects, do not require ionizing radiation and are usually not invasive.

However, several challenges compromise the diagnostic utility of MR imaging and limit its accessibility, particularly for operators without specialized training in the physics of MR imaging and protocol optimization. One essential issue is the impact of patient-induced factors on image quality. Variations in the static magnetic field (B₀) or the radiofrequency field (B₁), caused by patient anatomy or positioning, lead to signal non-uniformities and spatial inconsistencies that affect image quality, contrast and diagnostic reliability. Motion artifacts, whether voluntary or involuntary, introduce phase shifts and signal blurring that obscure critical anatomical structures, while flow-related disturbances, such as blood or cerebrospinal fluid motion, result in ghosting or other artifacts that further complicate image interpretation. These patient-related challenges also affect techniques such as fat suppression, which is essential for enhancing lesion visibility. Fat suppression often fails in regions with field inhomogeneities or off-resonance effects, leading to inconsistent fat signal elimination or complete signal voids in regions of intrinsic tissue motion, compromising diagnostic accuracy.

Compounding these technical challenges are the complexity of the user interface of conventional MR systems and the workflow, which requires operators to perform numerous manual adjustments to tailor the parameters of the protocols and MR imaging sequences to specific clinical scenarios. In addition, the operators do not get immediate feedback on the effects of the protocol adjustments with the consequence that only after the imaging scan is finished the result of the operator intervention is seen. All this making MR imaging predominantly a tool for highly trained specialists. This complexity is further exacerbated when trying to mitigate the afore-mentioned patient-specific issues such as motion, flow or field inhomogeneities, as these require additional parameter adjustments and expertise.

Despite these challenges, the primary diagnostic objective remains achieving optimal image quality in the clinically relevant region of interest while often a lower quality in non-critical areas can be accepted.

### SUMMARY OF THE INVENTION

From the foregoing it is readily appreciated that there is a need for an improved method of MR imaging that addresses the interplay of patient-induced artifacts, operational complexity, and the need for precise imaging of clinically relevant areas. It is thus an object of the invention to improve the diagnostic utility and accessibility of MR imaging while ensuring high-quality imaging across a broad range of clinical contexts.

In accordance with the invention, a method of MR imaging is disclosed. The method comprises the following steps:
a) acquiring MR imaging data from a patient's anatomy by executing at least one (pre-) scan to generate an image of the anatomy and to extract information including at least one of coil sensitivity distribution, B₀ field distribution, B₁ field distribution, spatial distribution of flow characteristics, spatial distribution of motion characteristics;
b) identifying an anatomic structure or region of interest within the imaged anatomy;
c) automatically adapting settings of an MR scanner used and/or parameters of an MR imaging sequence or protocol taking the extracted information into account for enhancing image quality or diagnostic utility for the identified anatomic structure or region of interest; and
d) acquiring further MR imaging data from the patient's anatomy using the MR imaging sequence or protocol with the adapted settings and/or parameters to generate one or more optimized images of the identified anatomic structure or region of interest.

The disclosed method represents an approach to enhancing the diagnostic utility and user-friendliness of MR imaging through an automated process for region-specific optimization.
The method begins with acquiring MR imaging data through a (pre-) scan of the patient's anatomy. This initial scan may be a pre-scan, meaning that it is conducted before the actual imaging or diagnostic scan. Its sole objective is to serve the calibration and optimization of the subsequent regular MR image acquisition. The pre-scan phase is not designed to generate a diagnostic image but rather to tune the scanner's settings for the individual patient and clinical question. However, in an alternative embodiment, the initial scan may be a regular scan constituting a part of the main imaging phase, during which diagnostic data is collected. The initial scan of the method of the invention serves a dual function: generating an initial image of the anatomy and extracting essential information about the imaging environment and the patient's physiological characteristics. Extracted information can include the coil sensitivity distribution, which maps the spatial sensitivity distribution of the RF coils used for MR signal acquisition, B₀ field distribution, which maps the static magnetic field across the imaged region to detect inhomogeneities caused by tissue susceptibility variations or external factors. Additionally, B₁ field distribution maps can be generated to assess the uniformity of the radiofrequency (RF) field, critical for ensuring consistent signal excitation and reception. The (pre-) scan can also capture the spatial distribution of flow characteristics, such as the movement of fluids like blood, and motion characteristics related to respiration, cardiac activity, or patient movement. This detailed characterization of the imaging conditions establishes a foundation for optimizing imaging and protocol parameters specific to the patient and region of interest.

Following the (pre-) scan, the method identifies an anatomic structure or region of interest within the imaged anatomy. This identification step may be performed interactively, allowing clinicians to pinpoint the area based on clinical expertise or imaging findings, or automatically, using advanced algorithms such as machine learning or image segmentation techniques informed by the clinical question at hand. To automatically repeat initially selected and dragged image structures based on a clinical request, there are various machine learning (ML) and computer vision techniques that can be incorporated in a learning phase. Convolutional Neural Networks (CNNs) or object detection models like YOLO disclosed in Redmon et al., You only look once: Unified, real-time object detection; Proceedings of the IEEE Conference on Computer Vision and Pattern Recognition (CVPR), 2016, pp. 779-788, Faster R-CNN disclosed in Ren, S et al., Faster R-CNN: Towards Real-Time Object detection with Region Proposal Networks; IEEE Transactions on Pattern Analysis and Machine Intelligence, vol. 39, no. 6, pp. 1137-1149, 2017, doi: 10.1109/TPAMI2016.2577031, hereby incorporated in their entirety, and Single shot multiBox detector (SSD), allow identifying objects in images and can be trained to recognize multiple object categories. Others are feature extraction methods, self-supervised learning for pattern detection, clustering and pattern matching techniques or Vision Transformers. The flexibility in this step ensures that the system is adaptable to various clinical scenarios. Interactive identification allows for user control in complex or ambiguous cases, while automatic methods enhance efficiency and reduce operator variability in standard situations.

Once the region of interest has been identified, the method automatically adapts the settings of the MR scanner used and/or the parameters of the MR imaging sequence or protocol, taking into account the information extracted during the (pre-) scan. Parameters such as repetition time (TR), echo time (TE), flip angle, and inversion time (TI) are optimized to enhance contrast and improve the signal-to-noise ratio for the targeted region. Spatial resolution is refined through adjustments to the field of view, matrix size, and slice thickness, while magnetic field uniformity is improved via B₀ shimming and RF field adjustments through B₁ shimming. Artifact reduction strategies are tailored based on the spatial distribution of flow and motion sensitivity detected in the (pre-) scan. These automatic adaptations ensure that the imaging protocol is dynamically tailored to the patient's specific anatomy and the clinical question, enhancing both image quality and diagnostic relevance.

The B₀ and/or B₁ field maps extracted from the MR imaging data of the (pre-) scan provide critical spatial information about the uniformity and variations of the magnetic and radiofrequency fields across the imaged anatomy. These maps are essential for adapting the scanner settings and/or parameters of the MR imaging sequence or protocol, ensuring optimal image quality, enhanced signal uniformity, and reduced artifacts.

For example, shimming adjustments, based on the B₀ map, can be applied for optimizing the homogeneity of the magnetic field. Automated algorithms can analyze the B₀ map to adjust shim coil setting, enhancing uniformity in the region of interest and minimizing associated artifacts. Shim coil settings can be also used to suppress anatomical regions not relevant for the diagnosis as selected. An advantage is improved motion characteristics. Fat suppression techniques, such as spectral fat saturation, are also sensitive to B₀ field variations. By using the B₀ map, the system can adapt the center frequency and bandwidth of RF pulses to achieve consistent fat suppression across the imaging volume.

The B₁ map indicates the spatial distribution of the radiofrequency field used for excitation and signal detection. Inhomogeneities in the B₁ field can lead to uneven excitation or signal reception, resulting in intensity and contrast variations across the image. B₁ shimming uses the information from the B₁ map to adjust the amplitudes and phases of RF signals delivered by different RF coil elements or antennas, thus improving RF field uniformity and reducing signal intensity variations. Flip angle corrections are another application, ensuring that the prescribed flip angle is achieved across the imaging volume, preserving both contrast and signal-to-noise ratio.

The B₀ and B₁ maps can be used in combination to optimize imaging protocols. In echo planar imaging (EPI), for example, the B₀ map helps to address geometric distortions. Especially at higher field strength, sequences like TSE, for example, benefit from B₁ shimming using the B₁ map to ensure consistent signal intensity.

By incorporating B₀ and B₁ maps into the imaging workflow, the MR imaging system dynamically adapts sequence and protocol parameters to the specific characteristics of the patient's anatomy and the region of interest. This leads to improved image quality, greater diagnostic utility, and reduced need for repeat scans, enhancing the overall efficiency and effectiveness of the MR imaging process.

The terminology "clinical question" used in the description of the invention shall be understood in the following context: typically patients are referred to an MR scan for a structured query or clinical question to guide decision-making focusing on diagnosing or understanding a health issue. Hereby a medical expert like a doctor or radiologist suspects a disease and uses the MR system to answer the clinical question. In case the clinical question is to detect a "suspected Hepatocellular Carcinoma (HCC)", as an example, the radiologist would use a standard T1 scan with injection of a contrast agent like Gadolinium to image the liver region. Hereby the radiologist is only interested in scanning the liver region. Through the selection of the anatomy of interest , for example by a simple select and drag mechanism, it is easy and intuitive to select the liver and spleen area maybe including potential lymph node areas to check for tumor spreads, leaving out clinical not relevant regions like the heart or the arms that may interfere with the scan - introducing motion and blood pulsation artifacts from the heart or suboptimal B₀ leading to distortions or fat suppression and B₁ shading. Hereby the pre-scan allows to identify regions of motion, flow, B₀, B₁ difficulties and preparation settings and the protocol settings are adapted for an artifact free image of the selected region of interest with respect to the clinical question.

The acquisition of spatial distribution (maps) of flow and motion characteristics within the imaged anatomy provides critical insight into regions where flow and motion are likely to occur. These maps can be used according to the invention as predictive tools, enabling the identification of areas prone to motion-related and flow-related artifacts. By understanding where within the anatomy these phenomena originate, MR imaging sequences and protocols can be adapted in a targeted manner to minimize their impact and improve the overall diagnostic value of the images.
Flow maps can indicate, e.g., the spatial distribution of fluid motion, such as blood flow in vessels or cerebrospinal fluid (CSF) movement. These maps provide detailed information on the velocity and direction of flow, enabling the identification of regions with high flow dynamics, such as major arteries, veins, or turbulent flow areas. Motion maps, on the other hand, reveal regions affected by physical motion, such as respiratory-induced movement of the chest, cardiac motion of the heart, or involuntary patient movement. These maps can highlight not only the areas directly affected by motion but also the regions downstream that are indirectly influenced by motion propagation.

The key advantage of these maps lies in their ability to localize the origin of motion or flow. For example, respiratory motion often originates in the diaphragm and propagates to adjacent organs, while blood flow artifacts are typically tied to major vessels. By pinpointing the origin of these effects, imaging protocols can be adapted to specifically target the source, thereby reducing their negative impact on image quality.

The quantitative determination of motion and flow within the imaged anatomy offers significant advantages in the context of MR imaging, particularly when adapting imaging sequences and protocols to optimize image quality and diagnostic accuracy. These advantages arise from the ability to objectively measure, localize, and understand the characteristics of motion and flow phenomena, enabling precise and targeted adaptations that mitigate artifacts and enhance diagnostic utility.

Flow maps quantitatively assessing flow in the anatomy of interest offer advantages particularly in the context of imaging vascular structures. In regions with high-velocity pulsatile flow, such as major arteries, ghosting artifact may overlay with the anatomy of interest, flow displacements and flow dephasing may be seen. Flow-sensitive sequences like phase-contrast MR imaging or velocity-encoded imaging can be optimized to accurately capture the dynamics. Conversely, in areas of slow or stagnant flow, the MR imaging sequence can be adapted to enhance sensitivity to subtle flow patterns, such as those in capillaries or small veins.

The ability to quantitatively map motion and flow also enables predictive modeling of their impact on image quality. By integrating motion and flow data into the imaging workflow, the system can simulate the expected artifacts for different sequence configurations and adapt protocols accordingly. For instance, in regions where motion artifacts are predicted to obscure critical anatomy, the system might prioritize higher temporal resolution or employ artifact-minimizing strategies such as parallel imaging or non-cartesian sampling techniques (like Propeller, radial or spiral sampling. Similarly, in regions with significant flow-related artifacts, flow-compensated sequences or alterations to gradient timings can be applied to suppress unwanted effects. With a spatial quantitative estimate of the flow, the B₀ shim settings can be optimized, suppressing vessels outside the anatomy of interest. The MR imaging sequence can be adapted by application of flow suppression pulses but also (semi-) automatic adaptations of the protocol using partial echo and/or flow compensation gradients. Flow compensation could be adapted to shorten echo times, adjusting flow compensation grades based on the quantitative flow estimate of the (pre-) scan.

Overall, the quantitative determination of motion and flow within the imaged anatomy advantageously enables a more precise and informed adaptation of MR imaging sequences and protocols. By providing objective, reproducible metrics, it enhances the ability to address artifacts, improves visualization of complex dynamics, and supports advanced diagnostic assessments. This approach ultimately leads to improved image quality, reduced scan times, and increased diagnostic confidence.

Using the information provided by the described motion and/or flow maps, MR imaging sequences and protocols can be adapted in several ways according to the invention. For motion-prone regions, e.g., the acquisition time point and acquisition window can be adapted automatically avoiding pulsatile or breathing motion, or motion compensation techniques can be employed, such as respiratory or cardiac gating/triggering, which synchronizes image acquisition with the motion cycle based on the detected quantitative motion/or flow map information, or switching to motion resistant sequences like Propeller, radial or spiral acquisition schemes. Navigator pulses, (intrinsic) k-space navigator, physiology sensors or motion correction algorithms can be used automatically to detect and correct motion artifacts in real time. For regions with significant flow dynamics, flow-compensated sequences can be selected automatically to counteract flow-induced phase shifts, or velocity-encoded imaging can be implemented to quantify and incorporate flow into diagnostic evaluations.

The integration of these maps into the imaging process is particularly powerful in guiding the customization of the field-of-view (FOV), slice orientation, and acquisition timing. For instance, slices can be oriented to avoid major motion artifacts or to align with flow paths, minimizing phase-encoding errors. Sequence timing can also be adjusted to occur during periods of minimal motion, such as specific phases of the respiratory or cardiac cycle.

This targeted approach to artifact prediction and sequence/protocol adaptation not only enhances image quality but also improves diagnostic utility. By localizing and addressing the origins of flow and motion, the method ensures clearer visualization of relevant anatomical structures and reduces the need for additional scans. This predictive and adaptive capability ultimately streamlines the imaging workflow, reduces patient discomfort, and increases confidence in diagnostic interpretations.

With the thus adapted, optimized parameters in place, the method of the invention acquires further MR imaging data from the patient's anatomy, focusing on the identified anatomic structure or region of interest. These images reflect the enhancements applied in the preceding steps, providing optimized visualization tailored to the diagnostic requirements. By concentrating on the region of interest with patient-specific adaptations, the method improves the clarity and diagnostic utility of the images while reducing unnecessary imaging of irrelevant areas, thereby enhancing workflow efficiency.

Moreover, the flow and motion sensitivity maps can also be used to improve the relevant B₀, B₁, coil sensitivity maps to improve image quality.

By combining (pre-) scan data analysis, flexible identification of the anatomy/region of interest, and dynamic adaptation of imaging parameters, the method of the invention ensures high-quality, patient-specific imaging. The flexibility to perform interactive or automatic region identification accommodates a broad range of clinical scenarios, making the method suitable for applications ranging from routine diagnostic imaging to specialized studies in oncology, cardiology, or neuroimaging. Overall, it reduces operator dependency, increases reproducibility, and improves diagnostic accuracy.

An MR imaging protocol within the meaning of the invention is a predefined set of one or more MR imaging sequences, parameters and instructions used to perform one or more specific MR imaging scans. The protocol defines in detail how the used MR imaging system operates to acquire imaging data and generate images tailored to the particular clinical question, anatomical region, or diagnostic requirement. Parameters of the MR imaging sequence, such as echo time, repetition time, flip angles, and gradient strengths can be adapted to account for patient anatomy, pathology, and potential artifacts. Parameters of the MR imaging protocol that can be adapted in accordance with the invention include: the field of view (FOV) defining the area to be covered by the MR scan which is critical for focusing on the identified anatomic structure or region of interest; the matrix size determining image resolution, with higher matrix sizes yielding finer details; slice thickness can be adjusted to improve resolution or minimize partial volume effects, while slice gap has to be optimized to minimize artifacts and ensure complete coverage of the region; the number of slices is adjusted based on the size of the anatomy or region of interest, and temporal resolution involves modifications to scan duration or frame rates for dynamic studies; contrast and weighting adjustments involve selecting the appropriate weighting type, such as T₁, T₂, proton density, or diffusion-weighted imaging, depending on the diagnostic goal; fat suppression techniques like STIR, Dixon, or spectral fat saturation are applied for enhanced tissue differentiation; parameters related to diffusion imaging, such as diffusion gradient strength and direction, are customized for specific protocols, and echo planar imaging (EPI) factors are adjusted for fast imaging in diffusion or functional studies; signal-to-noise ratio (SNR) and artifact reduction can be achieved by averaging, which increases SNR through scan repetition, and parallel imaging factors, such as GRAPPA or SENSE or CS SENSE (AI), which balance acquisition speed and SNR; shimming parameters can be adjusted to enhance B₀ field uniformity and reduce artifacts like flow and motion artifacts, while flow compensation minimizes motion artifacts in vessels or fluid regions; in case of severe motion switching to advanced techniques like Propeller, Radial or Spiral are possible options. Timing and scan workflow adjustments include triggering and gating, optimized for respiratory or cardiac cycles to minimize motion artifacts, and scan timing synchronization with physiological cycles or external stimuli; further hardware and system settings involve optimizing gradient strength and slew rates for resolution and scan speed, and tailoring RF pulse amplitude and shape for uniform excitation or specific tissue targeting; coil configurations are selected or adjusted to maximize sensitivity for the identified anatomic structure or region of interest.

In another embodiment, the method includes the step of collecting sensor data from the patient, leveraging additional inputs to refine the imaging process. The sensor data may include physiological sensor data, motion sensor data, magnetic field sensor data, or ultrasound sensor data, providing a comprehensive view of the patient's state and environment. This data is then used in combination with the information extracted from the (pre-) scan data to adapt the parameters of the MR imaging sequence or protocol, ensuring that the imaging process is tailored to the specific conditions of the patient and the identified anatomic structure or region of interest. Physiological sensor data, such as heart rate, ECG, respiratory rate, or oxygen saturation, are particularly valuable for imaging regions affected by physiological motion, such as the thorax or abdomen. These data enable the implementation of gating or triggering techniques, synchronizing image data acquisition with specific phases of the cardiac or respiratory cycles to minimize motion artifacts. Motion sensor data, collected, e.g., via accelerometers, gyroscopes, cameras or respiratory belts, provide real-time information on patient movement during the scan. This data is useful for identifying voluntary or involuntary motion, allowing the method to dynamically adjust imaging parameters, automatically select appropriate imaging protocols or employ motion-correction algorithms during reconstruction. Magnetic field sensor data can be used to monitor the local magnetic field environment and detect field fluctuations caused by external factors or patient anatomy. This information enables the method to fine-tune shimming mechanisms, ensuring uniformity in the B₀ field. Accurate magnetic field mapping derived from sensor data helps reduce artifacts such as distortions or signal voids, particularly in regions with high susceptibility gradients. Ultrasound sensor data provide additional anatomical and functional insights, particularly for dynamic imaging or complex regions where direct MR visualization may be challenging. For example, ultrasound guidance can assist in identifying anatomic structures in real-time, such as vascular pathways or moving organs, and complement MR imaging to improve targeting and alignment of the imaged anatomy. Ultrasound-derived motion or structural information can also inform adaptations to imaging parameters, such as optimizing acquisition timing or improving spatial resolution in the respective areas of interest. By integrating these diverse sensor data streams, the method ensures that adaptations of parameters and/or settings to the MR imaging sequence or protocol or MR scanner hardware account for patient-specific physiological, anatomical, and environmental conditions. These adaptations may include adjustments to temporal resolution, gradient strength, echo times, field of view, or contrast weightings, all tailored to enhance the imaging of the identified anatomic structure or region of interest.

In another embodiment, the method includes the step of adapting the settings of the MR scanner itself, with specific adjustments to its hardware components to enhance imaging performance for the identified anatomic structure or region of interest. This adaptation involves tuning elements of the MR imaging system, such as gradient coils, radiofrequency (RF) coils, and shimming mechanisms, to address the unique imaging requirements of the selected anatomy or region. RF coils or antennas, responsible for transmitting and receiving the MR signal, can be selected in specific configurations (e.g., phased-array or surface coils) that maximize the signal-to-noise ratio (SNR) for the target anatomy. The method may prioritize coil elements that provide the best coverage and sensitivity, ensuring uniform signal reception and minimizing noise. Automatic shimming adjustments can be performed to fine-tune the homogeneity of the static magnetic field (B₀) in the vicinity of the selected anatomy (organ) or region of interest. Improved field uniformity reduces artifacts such as signal dropouts or distortions, particularly in regions with complex anatomy or high susceptibility differences, such as the brain or musculoskeletal joints. By incorporating these hardware-level adjustments, the method ensures that the MR scanner operates in a manner optimized for the specific anatomical and diagnostic requirements. Automating these hardware adjustments further reduces the need for operator intervention and technical expertise, enabling consistent and reliable performance across different operators and clinical settings. This embodiment complements the other optimization steps in the method of the invention, providing a comprehensive approach to achieving high-quality, region-specific MR imaging tailored to the clinical question.
The concept of an "uncertainty map" in the context of the invention can be considered as a representation that describes the expected level and spatial distribution of residual artifacts in the reconstructed image due to motion and/or flow, even after the adaptation of sequence and protocol parameters. This map is a valuable tool for identifying areas where image quality may still be compromised, guiding diagnostic interpretation, and potentially prompting further adaptive strategies or additional scans. To derive an uncertainty map, several steps are typically involved, leveraging both quantitative data on motion and flow and knowledge of the adapted imaging sequence and protocol parameters. In a possible embodiment, the quantitative measurements of B₀, B₁, RF coil sensitivity, motion and/or flow can be input into a predictive model that simulates the impact on the reconstructed image. The model considers the characteristics of the adapted imaging sequence or protocol. The output of the predictive model is an uncertainty map, which represents the residual artifact potential as a function of spatial location. Each voxel in the uncertainty map can be assigned a value indicating the expected artifact level, with higher values corresponding to regions more likely to exhibit residual blurring, ghosting, or signal distortions. The uncertainty map can be also adjusted automatically taking any system and scanning/protocol adaptations into account. The map can be displayed as an overlay on the reconstructed image, providing a visual guide to regions where interpretation should be approached with caution.
The uncertainty map has several practical applications. During diagnostic interpretation, it alerts clinicians to areas where artifact-related uncertainty may impact diagnostic confidence, prompting a more nuanced evaluation or the consideration of additional imaging. The uncertainty map can also guide further adaptations in the MR imaging sequence or protocol. For example, regions with high uncertainty may prompt the use of alternative sequences, refined motion correction algorithms, or additional acquisitions with different parameters. Additionally, the map supports research into improving motion and flow compensation techniques by highlighting regions where current strategies are insufficient.

In some embodiments, the step of identifying the anatomic structure of region of interest includes:
- presenting the generated image to a user via a user interface;
- receiving, via the user interface, a selection input from an operator by pointing at an arbitrary position and/or dragging between arbitrary positions within the image of the anatomy; and
- processing the selection input and the image of the anatomy to identify the anatomic structure or region of interest corresponding to the position selected within the image, wherein the identification is performed by automated object selection using an image segmentation algorithm or machine learning model trained to recognize anatomic structures. In this variant, the image generated from the MR imaging data of the (pre-) scan is presented to the operator via the user interface. The user interface is designed to provide visualization of the anatomical structures, enabling the operator to review the image and identify regions of clinical interest. This interface provides an intuitive platform for interaction to the operator. The operator then interacts by providing a selection input, pointing at a position at discretion, marking a region by dragging over it within the image or hovering over a region. This interaction may be facilitated by a pointing device, such as a mouse or touch interface, and allows the operator to identify a specific anatomical region of interest. The simplicity of this interaction reduces the expertise needed to operate the MR system, making it accessible to a broader range of users. Following the operator input, the method processes the selection and the image to identify the anatomic structure or region of interest corresponding to the pointed position. This identification can then be performed automatically using advanced image segmentation algorithms or machine learning models that have been trained to recognize anatomical features. These models analyze the spatial and textural characteristics of the image in conjunction with the input location to delineate the boundaries of the selected structure/organ or region of interest. By leveraging automated segmentation, the method ensures accuracy and consistency in the identification of the relevant anatomy, independent of operator expertise.

By integrating fully automated or user-guided automated identification of the relevant anatomy structures or region of interest and sequence/protocol optimization, the method reduces the complexity of MR imaging, making it accessible to a broader range of users, including non-specialists. The interactive user interface simplifies operator input, requiring only basic anatomical knowledge to achieve precise region-specific imaging. Furthermore, the use of machine learning and segmentation algorithms that are known in the art as such for automated object selection ensures accurate and reproducible identification of the relevant anatomy to be imaged, thus minimizing operator-dependent variability. The automatic adaptation of imaging parameters enhances diagnostic confidence by focusing imaging resources on the region of interest while suppressing irrelevant areas, leading to improved image quality in clinically relevant zones. This targeted approach significantly increases the efficiency of MR imaging workflows.

In an additional embodiment, the results of the artificial intelligence (AI) based automatic selection of the anatomic structure or region of interest can be saved for future use. Once the method identifies the anatomic structure or region of interest corresponding to the operator's input, this information, including the specific anatomical delineation, can be stored in a database. This stored data is preferably indexed alongside metadata such as the clinical question and patient information. In subsequent examinations involving equivalent clinical questions or similar anatomical regions, the saved information on the anatomy of relevance can be retrieved and applied automatically. This retrieval facilitates rapid setup of imaging parameters, significantly reducing the time and effort required for protocol optimization. By leveraging previously identified anatomical patterns and optimized imaging strategies, the method ensures consistency across similar (e.g., follow-up) examinations, enhancing diagnostic reproducibility and workflow efficiency. Additionally, the stored data can contribute to refining the machine learning models over time, as repeated application and feedback from similar clinical scenarios enable the algorithm to learn and improve its accuracy and generalizability.

In another embodiment, the method incorporates the step of selecting a MR imaging sequence or protocol in a (semi-) automated or manually executed step from a list of available MR imaging sequences or protocols suited to image the identified anatomic structure or region of interest for diagnostic purposes taking the extracted information into account for the planning and the optimization of the sequence. Once the method has identified the anatomic structure or region of interest, the method evaluates the imaging needs of the selected anatomy or region, such as spatial resolution, contrast mechanisms, and sensitivity to specific tissue characteristics of the identified anatomy. The method can then match these requirements to a catalogue of pre-configured imaging sequences or protocols that are optimized for various anatomical regions and clinical questions. This catalogue includes, e.g., a diverse set of sequences designed for different diagnostic purposes, such as T₁-weighted, T₂-weighted, diffusion-weighted imaging (DWI), or sequences optimized for detecting specific pathologies like tumors, or vascular abnormalities. The selection process leverages a decision-making algorithm that considers factors such as the anatomical location, clinical question, and patient-specific characteristics, including prior imaging findings or known contraindications.

In yet another embodiment, the method includes the step of automatically adapting the settings of an image reconstruction algorithm used to generate the one or more optimized images, wherein the adaptation is configured to take the extracted information and/or adapted scanner settings and/or sequence/protocol parameters into account to improve at least one of: image quality, artifact suppression, image homogeneity, image contrast, image resolution, for the identified anatomic structure or region of interest. The adaptation process leverages the specific characteristics of the identified anatomy or region selected, taking into account, e.g., its size, location, type of tissue and diagnostic requirements. For example, if the selected anatomy or region corresponds to a small anatomical feature requiring fine detail, the algorithm may prioritize higher spatial resolution in the respective region. For regions requiring contrast differentiation, the reconstruction settings may emphasize signal-to-noise ratio improvements or optimize the balance between T₁ and T₂ contrast mechanisms. Additionally, for areas prone to artifacts - such as motion or susceptibility artifacts - the method may apply advanced artifact reduction techniques, including motion correction algorithms, parallel imaging reconstruction, or susceptibility artifact mitigation strategies. This automatic adaptation ensures that the reconstructed images are optimized for the specific diagnostic needs of the identified anatomic structure or region of interest. It reduces the need for operator intervention in fine-tuning reconstruction settings, further streamlining the imaging workflow.

In another embodiment, the method includes the step of identifying anatomic structures or regions that are not relevant for clinical diagnosis based on the operator's selection input. This step is integral to optimizing imaging efficiency and diagnostic focus by selectively excluding or deprioritizing regions that do not contribute to the clinical question at hand. The identification of such irrelevant regions or structures can be achieved automatically or through a combination of user interaction and automated analysis. For example, the method can automatically identify those regions that fall outside the identified anatomic structure or region of interest as being not relevant for the clinical diagnosis. Once irrelevant structures or regions are identified, the method applies this information during subsequent setting of the MR scanner hardware as well as subsequent imaging and processing steps. For imaging, the method can deprioritize or exclude these regions by adjusting the field of view, slice coverage, or spatial resolution to allocate imaging resources primarily to the clinically relevant regions. This targeted approach enhances imaging efficiency by reducing scan time and computational overhead without compromising diagnostic accuracy. During image processing, irrelevant regions can be suppressed or masked to streamline visualization and interpretation by excluding or reducing image features. This selective processing minimizes operator distraction by providing clearer, more focused images for interpretation.

In a possible refinement of the method, artifacts originating from clinically irrelevant regions can be addressed, ensuring their suppression in a targeted and efficient manner. While the not relevant regions may be deprioritized or excluded during imaging or processing, they can still generate artifacts that propagate into the clinically relevant anatomic structure or region of interest, potentially compromising diagnostic quality. This additional step ensures that artifacts from non-relevant regions are mitigated. In the image reconstruction and post-processing stages, spatial masking and signal filtering techniques can be applied to limit the influence of artifacts from irrelevant areas. Furthermore, the method may incorporate machine learning models trained to predict and suppress specific artifact patterns based on patient-specific data collected during the (pre-) scan. These models can identify artifact-prone regions and preemptively adjust imaging or processing parameters to minimize their impact in the identified anatomic structure or region of interest.

In a further refined embodiment, the (pre-) scan employs a multi-echo Dixon technique, which may employ different flip angles and which may include the simultaneous collection of sensor data (see above) to:
- extract the B₀ field distribution within the selected anatomic structure or region of interest as well as in the non-relevant region,
- extract the B₁ field distribution within the selected anatomic structure or region of interest as well as in the non-relevant region,
- extract the coil sensitivity distribution within the selected anatomic structure or region of interest as well as in the non-relevant region, and/or
- determine the spatial distribution of motion and/or flow sensitivity within the selected anatomic structure or region of interest as well as in the non-relevant region. The multi-point Dixon technique is a versatile and robust method for acquiring multiple echo signals at distinct echo times, allowing for detailed characterization of field inhomogeneities and the spatial distribution of motion and flow sensitivity. By analyzing the phase and magnitude information from these echoes, B₀ and B₁ field distributions, as well as motion and flow sensitivity maps, can be derived with high accuracy. The multi-point Dixon method captures the phase evolution caused by differences in local magnetic field environments. This phase evolution is sensitive to both static field inhomogeneities (B₀ variations) and temporal variations such as flow or motion effects. Additionally, the magnitude data can provide complementary information about RF field uniformity (B₁) and signal intensity variations. The B₁ field distribution can be derived, e.g., by analyzing the magnitude of the signal across images with different flip angles for example. Inhomogeneities in the RF field result in spatially varying flip angles, which influence signal intensity. The motion and flow sensitivity distribution can be derived by examining phase variations that deviate from the predictable phase evolution caused by B₀ inhomogeneities. Flowing spins experience velocity-dependent phase shifts due to gradients applied during the imaging sequence, and these shifts are captured in the phase differences between echoes. By modeling these phase shifts using velocity-encoding equations, the spatial distribution of flow sensitivity can be quantified, including velocity magnitude and direction. Motion sensitivity is similarly derived by identifying phase inconsistencies that correlate with known motion patterns, such as respiratory or cardiac cycles. Advanced algorithms, including temporal filtering or machine learning approaches, can further enhance the detection of motion-related phase variations. Combining these analyses, the multi-point Dixon method can produce detailed maps of B₀ and/or B₁ field distributions and motion/flow sensitivity. These maps can be used to adapt imaging parameters dynamically as described above. In summary, the multi-point Dixon technique leverages the phase and magnitude information from multiple echoes to provide a comprehensive understanding of the magnetic and dynamic characteristics of the imaged region.
   Integrated k-space motion sensing navigators can be used to further augment the (pre-) scan by providing real-time detection of patient motion. These navigators, embedded within the k-space acquisition process, utilize MR signal data from one or more RF coil elements to track motion with high sensitivity and spatial specificity. This capability is particularly beneficial for identifying involuntary motion, such as respiration or cardiac pulsation, as well as subtle shifts in patient positioning.

Determining B₀ and B₁ field maps, coil sensitivity maps as well as motion and flow sensitivity maps, in both the identified anatomic structure or region of interest and the non-relevant regions offers significant advantages for enhancing image quality, minimizing artifacts, and improving diagnostic utility. The inclusion of the non-relevant regions in this analysis ensures a comprehensive understanding of the entire imaging field and its potential influence on the region of interest. The primary advantage is the ability to identify sources of artifacts originating in the non-relevant regions that may propagate into the region of interest. For example, magnetic field inhomogeneities in non-relevant areas, can cause susceptibility artifacts that extend into the region of interest, degrading image quality. By determining the B₀ field distribution across the entire field of view, shimming adjustments can be optimized not only for the region of interest but also to mitigate these external influences. This approach reduces geometric distortions, signal loss, and phase inconsistencies that could otherwise obscure diagnostically critical structures.

In particular, assessing motion and flow sensitivity in non-relevant regions provides valuable insights into potential sources of dynamic artifacts. For example, respiratory or cardiac motion originating outside the region of interest can induce phase shifts or ghosting artifacts that overlap with the region of interest, reducing diagnostic clarity. Similarly, high-velocity flow in major vessels outside the targeted region can cause flow-related artifacts that propagate into the imaged area. By determining motion and flow sensitivity in non-relevant regions, the imaging sequence or protocol can be adapted to address these issues proactively. Strategies such as gating, navigator pulses, or flow-compensated sequences can be applied to suppress artifacts originating from outside the region of interest, ensuring that these effects do not compromise the image quality of the targeted area.

Moreover, including non-relevant regions in the analysis supports a holistic optimization of the imaging protocol. For example, field-of-view adjustments, slice positioning, and gradient settings can be tailored based on the combined insights from the relevant and non-relevant regions. This comprehensive approach ensures that imaging parameters are optimized for the entire field, reducing the likelihood of artifacts that require repeat scans or additional post-processing.

In another embodiment, the extraction of the B₀ and/or B₁ field distribution, coil sensitivity distribution and/or the determination of the spatial distribution of motion and/or flow sensitivity is performed using artificial intelligence, such as a machine learning method trained to analyze the MR imaging data of the (multi-echo Dixon) (pre-) scan and/or the sensor data. This approach enhances the accuracy, speed, and robustness of the extraction. The multi-echo Dixon (pre-) scan generates a rich dataset containing phase and magnitude information across multiple echoes, which encodes the effects of magnetic field inhomogeneities, RF field variations, and dynamic phenomena such as motion and flow. Sensor data, such as motion tracking or physiological measurements, may further complement this dataset by providing additional temporal and spatial context for motion and flow dynamics. A machine learning model trained on a diverse set of annotated (pre-) scan MR imaging data (optionally in combination with field/physiological/motion sensor data as described above) can learn to recognize complex patterns in these data, facilitating efficient and precise extraction of B₀ and B₁ maps and motion/flow sensitivity distributions.

The artificial intelligence (AI) can employ supervised, unsupervised, or hybrid learning approaches. For B₀ extraction, the machine learning algorithm learns to identify phase evolution patterns caused by static field inhomogeneities and correlates them with known B₀ field variations from the training data. For B₁ mapping, the algorithm interprets intensity variations in magnitude data, using a learned model of the relationship between signal amplitude, flip angle, and RF field strength. Motion and flow sensitivity distributions are determined by identifying phase inconsistencies or velocity-dependent phase shifts across echoes, optionally augmented by motion-specific features extracted from the sensor data. A significant advantage is the ability of artificial intelligence to integrate and interpret multimodal data. By combining MR imaging data from the multi-echo Dixon (pre-) scan with sensor inputs, a more comprehensive determination of motion and flow characteristics is achieved. Hereby smart k-space subsampling of the different gradient echoes, continuous physiology or B₀ monitoring as an example in combination with a GAN (generative adversarial network) may provide a means to efficiently reduce scan time of the (pre-) scan while maintaining high accuracy of the extracted information. This integration improves the reliability of motion/flow sensitivity maps and allows for more precise localization of artifact sources. For instance, the artificial intelligence model can predict not only where motion artifacts are likely to occur but also their periodicity, enabling tailored gating or navigator strategies.

In yet another embodiment, at least one of the steps of automatically adapting parameters of the MR imaging sequence or protocol, selecting the MR imaging sequence or protocol, automatically adapting the settings of the image reconstruction algorithm, automatically adapting settings of the MR scanner is performed using artificial intelligence, comprising a machine learning method trained to analyze the B₀ and/or B₁ field distribution and/or the coil sensitivity distribution and/or the spatial distribution of motion and/or flow sensitivity and/or the sensor data. This embodiment leverages artificial intelligence to perform automated, data-driven adaptations of MR imaging sequences, protocols, reconstruction algorithms, and scanner settings. Artificial intelligence, specifically machine learning, is employed to automate critical decision-making steps in the MR imaging workflow in an automatic or semi-automatic way. These steps include adapting parameters of the MR imaging sequence or protocol, selecting an appropriate sequence or protocol, adjusting the settings of the image reconstruction algorithm, and configuring the settings of the MR scanner. The machine learning model is trained to analyze B₀ and B₁ field distributions, spatial distributions of motion and flow sensitivity, and/or sensor data, enabling precise and adaptive optimization tailored to the patient and specific imaging requirements (according to the clinical question) to avoid artifacts and to obtain an optimized image quality. For example, B₀ field distribution maps can guide the machine learning model to suggest shimming adjustments or recommend sequences less sensitive to field variations, such as spin echo over gradient echo. Similarly, B₁ field distribution maps indicate RF field uniformity, allowing the artificial intelligence to optimize flip angles, excitation pulse design, or B₁ shimming settings to ensure consistent signal excitation and reception. Motion and flow sensitivity distributions provide spatial and temporal information about regions affected by motion or flow dynamics. The artificial intelligence model, trained on annotated datasets of such distributions, can predict their impact on image quality and recommend adaptive strategies. For instance, it might adjust acquisition timing to align with motion-free intervals, select gating techniques, or implement flow-compensated sequences for areas with significant flow-related artifacts. Sensor data, such as respiratory and cardiac signals, further enhance the model's ability to account for patient-specific dynamics, enabling suitable protocol adjustments. The artificial intelligence can further be used to optimize the settings of the image reconstruction algorithm. Using input from the B₀ and B₁ maps, the artificial intelligence can adapt reconstruction parameters such as spatial resolution, noise reduction thresholds, and artifact suppression settings. For example, in regions with high B₀ inhomogeneities, the model may enhance phase correction algorithms to mitigate distortion, while in areas with significant motion sensitivity, it might implement advanced motion correction techniques during reconstruction. The artificial intelligence-guided adaptation extends to the MR scanner hardware itself. By analyzing field distributions and motion/flow sensitivity maps, the artificial intelligence can optimize gradient coil settings, RF pulse amplitudes, and timing parameters to achieve the best balance between image quality, scan efficiency, and patient comfort. One of the key advantages of this artificial intelligence-driven approach is its ability to consider the complex interactions between multiple factors simultaneously. Traditional optimization methods often address these parameters in isolation, potentially missing subtle dependencies that affect overall image quality. In contrast, a machine learning model trained on diverse datasets can identify patterns and correlations across B₀ and B₁ distributions, motion/flow characteristics, and sensor data, enabling holistic optimization. This results in more accurate and reliable imaging protocols, particularly in challenging scenarios such as imaging near air-tissue interfaces, in patients with irregular motion patterns, or in anatomically complex regions. By automating the adaptation and selection processes, the artificial intelligence can significantly reduce the time required for manual adjustments and protocol configuration. This not only streamlines the imaging workflow but also minimizes operator variability, ensuring consistent and reproducible results across patients and imaging sessions.

In a further embodiment, the invention introduces the concept of a digital twin of the patient's anatomy, a virtual, data-driven representation created from the acquired MR imaging data and the identified anatomic structure or region of interest. In the context of the invention, the digital twin serves as a computational model that mimics the specific anatomical and physiological characteristics of the patient, reflecting their unique geometry, tissue composition, and imaging properties. This digital twin is constructed using advanced computational techniques that combine the initial MR imaging data with biophysical modeling, incorporating information such as tissue-specific relaxation properties, susceptibility characteristics, and spatial distributions of the magnetic (B₀) and radiofrequency (B₁) fields. The primary function of the digital twin is to provide a simulation environment for testing and visualizing imaging parameter adaptations before they are applied in the actual imaging. The adapted parameters of the MR imaging sequence or protocol, including sequence timing, gradient adjustments, field of view, RF pulse settings, MR scanner configurations etc., are applied to the digital twin. Through computational simulation, the effects of these adaptations on the virtual anatomy are analyzed, generating a predictive visualization of expected improvements in image quality, resolution, artifact suppression, or diagnostic utility for the ROI. This simulation-based visualization is presented to the operator via the user interface, offering a detailed preview of the anticipated results. By allowing the operator to assess the potential effectiveness of the adapted parameters, the method provides an opportunity to confirm that the proposed adjustments align with the clinical objectives before execution. This reduces the need for iterative trial-and-error adjustments during the actual MR scanning session, streamlining the imaging process and enhancing confidence in the planned protocol.

In a possible variant of the method, the digital twin becomes a tool for interactive parameter refinement or protocol adjustments. To this end, the method includes providing a control interface that enables the operator to modify the adapted parameters based on clinical judgment or specific diagnostic requirements. These operator-modified parameters are applied to the digital twin in a second round of simulation. The updated simulation results are used to generate a refined visualization, which is again presented to the operator. This iterative process allows the operator to fine-tune imaging parameters dynamically, leveraging both automated optimizations and operator input to achieve an optimal balance of image quality, diagnostic value, and scanning efficiency.

Moreover, the method may use the control interface to propose a certain imaging sequence or imaging protocol to the user and visualize the imaging results that can be expected using this particular sequence or protocol according to simulation. The digital twin in this method not only replicates the patient's anatomy but also incorporates dynamic simulation capabilities that take into account biophysical and imaging system-specific factors. For example, it may simulate the effects of B₀ and B₁ field inhomogeneities, motion, or flow within the patient's anatomy for different available MR imaging sequences and protocols, providing a highly realistic prediction of how the respective sequence or protocol will perform. This predictive capability allows for a comprehensive evaluation of the MR imaging sequence or protocol, ensuring that all relevant factors, including artifact suppression and signal uniformity, are addressed. The user can then decide which sequence or protocol to choose in a more targeted and informed manner.

The method of the invention described thus far can be carried out by means of a computer in a clinical MR imaging environment. The computer may be a processing unit operatively connected to an MR scanner. The MR scanner may include at least one main magnet coil for generating a main magnetic field within an examination volume, a number of gradient coils for generating switched magnetic field gradients in different spatial directions within the examination volume, at least one RF coil for generating RF pulses within the examination volume and/or for receiving MR signals as MR imaging data from a body of a patient positioned in the examination volume, with the processing unit controlling the temporal succession of RF pulses and switched magnetic field gradients based on an MR imaging sequence or protocol. The processing unit may further be configured to reconstruct MR images from the MR imaging data acquired by the MR scanner. In possible embodiments, separate computers may be used for controlling the MR scanner and for reconstructing MR images from the acquired MR imaging data respectively. The method of the invention can be implemented by a corresponding programming/software of the processing unit and/or reconstruction unit of the MR imaging system.

The method of the invention can be advantageously carried out in most MR environments in clinical use at present. To this end it is merely necessary to utilize a computer program by which the computer to be used is controlled such that it performs the above-explained method steps of the invention. The computer program may be present either on a data carrier or be present in a data network so as to be downloaded for installation in the computer, e.g. the control computer of an MR scanner.

### BRIEF DESCRIPTION OF THE DRAWINGS

The enclosed drawings disclose preferred embodiments of the present invention. It should be understood, however, that the drawings are designed for the purpose of illustration only and not as a definition of the limits of the invention. In the drawings:
Figure 1 shows an MR imaging system for carrying out the method of the invention;
Figure 2 illustrates an embodiment of the method of the invention as a flow chart;
Figure 3 schematically illustrates the collection and use of patient-individual information and its use for optimizing the acquisition of a diagnostic MR image.

### DETAILED DESCRIPTION OF THE EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

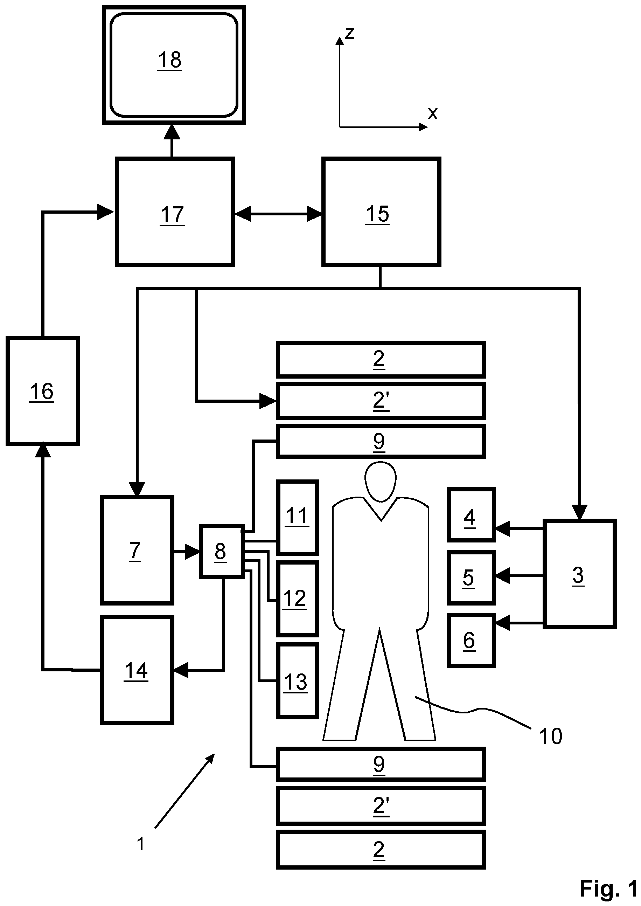

With reference to Figure 1, an MR imaging system 1 is shown. The MR imaging system 1 includes an MR scanner comprising superconducting or resistive main magnet coils 2 such that a substantially uniform, temporally constant main magnetic field B₀ is created along a z-axis through an examination volume. The scanner further comprises a set of (1^{st}, 2^{nd}, and - where applicable - 3^{rd} order) shimming coils 2', wherein the current flow through the individual shimming coils of the set 2' is controllable for the purpose of minimizing B₀ deviations within the examination volume.

A magnetic resonance generation and manipulation system applies a series of RF pulses and switched magnetic field gradients to invert or excite nuclear magnetic spins, induce magnetic resonance, refocus magnetic resonance, manipulate magnetic resonance, spatially and otherwise encode the magnetic resonance, saturate spins, and the like to perform MR imaging.

More specifically, a gradient pulse amplifier 3 applies current pulses to selected ones of whole-body gradient coils 4, 5 and 6 along x, y and z-axes of the examination volume. A digital RF frequency transmitter 7 transmits RF pulses or pulse packets, via a send/receive switch 8, to a body RF coil 9 to transmit RF pulses into the examination volume. A typical MR pulse sequence is composed of a packet of RF pulse segments of short duration which taken together with each other and any applied magnetic field gradients achieve a selected manipulation of nuclear magnetic resonance. The RF pulses are used to saturate, excite resonance, invert magnetization, refocus resonance, or manipulate resonance and select a portion of a body 10 positioned in the examination volume. The MR signals are also picked up by the body RF coil 9.

For generation of MR images of limited regions of the body 10 by means of parallel imaging, a set of local array RF coils 11, 12, 13 are placed contiguous to the region selected for imaging. The array coils 11, 12, 13 can be used to receive MR signals induced by body-coil RF transmissions.

The resultant MR signals are picked up by the body RF coil 9 and/or by the array RF coils 11, 12, 13 and demodulated by a receiver 14 preferably including a preamplifier (not shown). The receiver 14 is connected to the RF coils 9, 11, 12 and 13 via send/receive switch 8.

A control computer 15 constitutes a processing unit controlling the current flow through the shimming coils 2' as well as the gradient pulse amplifier 3 and the transmitter 7 to generate any of a plurality of MR pulse sequences, such as echo planar imaging (EPI), echo volume imaging, gradient and spin echo imaging, fast spin echo imaging, and the like in conformity with a pre-determined examination protocol. For the respective examination protocol, the receiver 14 receives a single or a plurality of MR data lines in rapid succession following each RF excitation pulse. A data acquisition system 16 performs analog-to-digital conversion of the received signals and converts each MR data line to a digital format suitable for further processing. In modern MR scanners the data acquisition system 16 is a separate computer which is specialized in acquisition of raw image data. The examination protocol determines the imaging sequences, i.e. the MR pulse sequences including the frequency and the temporal succession of radio-frequency pulses and/or magnetic field gradients to be activated. The parameters included in the examination protocol thereby characterize the MR image to be acquired, in particular with regard to location, orientation and size of the field of view as well as the contrast-weighting.

Ultimately, the digital raw image data is reconstructed into an image representation by a reconstruction unit 17 which applies a Fourier transform or other appropriate reconstruction algorithms, such as SENSE or SMASH. The MR image may represent a planar slice through the patient, an array of parallel planar slices, a three-dimensional volume, or the like. The image is then stored in an image memory where it may be accessed for converting slices, projections, or other portions of the image representation into appropriate format for visualization, for example via a video monitor 18 which provides a man-readable display of the resultant MR image.

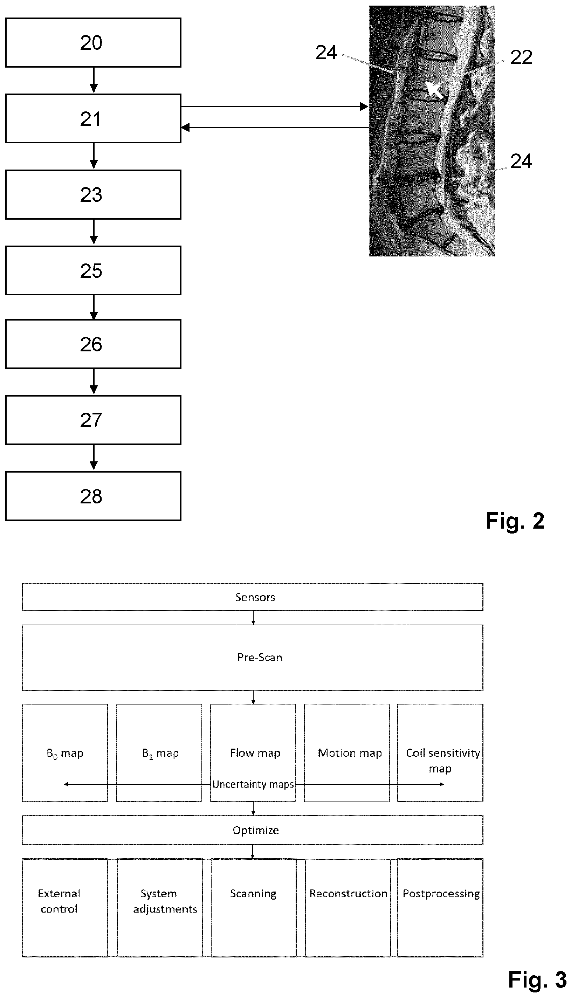

The method of the invention is a systematic approach to enhancing MR imaging through AI-based automation, patient-specific optimization, and interactive operator engagement. Referring to the flow chart in Figure 2 and with reference to Figure 3 the method is further detailed.

Initial data acquisition in step 20: MR imaging data is acquired from the patient's anatomy using for example a multi-point Dixon pre-scan to generate an initial image, serving as the baseline for further processing, and to extract information including a coil sensitivity map, B₀ field distribution (B₀ map), B₁ field distribution (B₁ map), spatial distribution of flow characteristics (flow map), spatial distribution of motion characteristics (motion map). This extraction is supported by simultaneously collected sensor data, such as physiological sensor data (ECG, breathing sensor), motion sensor data, and magnetic field sensor data providing a comprehensive view of the patient's state and environment. The uncertainty maps are determined in this step as well are representations that describe the expected level and spatial distribution of residual artifacts and errors due to B₀ and B₁ inhomogeneity, coil sensitivity, motion and/or flow. The uncertainty maps may also indicate uncertainties in the respective maps. The flow and motion maps may for example indicate uncertainties in the B₀ and B₁ maps, motion-induced uncertainties in the coil sensitivity map may cause uncertainties in the B₀ and B₁ maps as well.

Presentation and input in step 21: The generated image is displayed to the operator via a user interface (using, e.g., video monitor 18 in Figure 1). The operator provides a selection input by pointing at an arbitrary position within the image to identify an anatomic structure of clinical interest. This is illustrated in the example image in Figure 2 showing a section of the patient's spine. A mouse pointer 22 is moved over the spine region and the operator selects this anatomic structure by either continuously pushing the mouse button while moving the cursor, or by hovering over the region or by selection of certain points forming a predetermined geometry.

Automated structure identification in step 23: Based on the operator's input, the system processes the image to identify the anatomic structure of interest (the spine in the example) using AI (machine learning algorithms known as such in the art). The anatomic structure of interest is delineated from the surrounding tissue (tissue to the left and right of the spine in the depicted example image). The boundaries (indicated by lines 24) of the anatomic structure of interest are determined by the automated AI-based object selection. Object selection using machine learning techniques allows to identify, segment and select specific anatomy of interest in the MR images. Methods like object detection models, active learning models, wherein a user can refine the selection like GrabCut according to Rother et al., GrabCut: Interactive foreground extraction using iterated graph cuts, ACM Trans. Graph. 23(3), 2004, 309-314, hereby incorporated by reference in its entirety, are examples.

Parameter adaptation in step 25: Imaging parameters, including sequence settings, protocol adjustments, and scanner configurations, are automatically adapted using a machine learning method trained to analyze the B₀ and B₁ field distribution data, the coil sensitivity data as well as motion and flow characteristics determined in the (pre-) scan to optimize image quality and diagnostic utility for the selected anatomy. Therein, the uncertainty maps guide the adaptations, e.g. regions with high uncertainty may prompt the use of alternative sequences, refined motion correction algorithms, or additional acquisitions with different parameters.

Simulation via digital twin in step 26: A digital twin of the patient's anatomy is generated, reflecting the identified anatomic structure (the spine in the example of Figure 2). The effects of the adaptations performed in step 25 are simulated using the digital twin, producing a visualization of expected improvements in image quality and diagnostic utility, which is presented to the operator (not depicted).

Interactive refinement in step 27 (optional): The operator can modify the adapted parameters via a control interface (not depicted). The modified parameters are applied to the digital twin, and an updated simulation is presented, enabling iterative refinement before execution of the actual imaging step.

Optimized data acquisition and reconstruction in step 28: The result of the optimization in steps 25-27 are applied to control the MR scanner for the final MR imaging accordingly, namely to adjust system settings, perform the actual diagnostic MR scan. Moreover, reconstruction of the final MR image and, as an option, any postprocessing of the reconstructed MR image is performed in accordance with the adapted/optimized parameters derived from the pre-scan data to generate one or more optimized images of the identified anatomic structure.

In some embodiments at least one of the steps of automatically adapting parameters of the MR imaging sequence or protocol, selecting the MR imaging sequence or protocol, automatically adapting the settings of the image reconstruction algorithm, automatically adapting settings of the MR scanner is performed using artificial intelligence, comprising a machine learning method trained to analyze the B₀ and/or B₁ field distribution and/or the coil sensitivity distribution and/or the spatial distribution of motion and/or flow sensitivity and/or the sensor data.

The (semi-) automated optimization scheme of the invention, which leverages B₀ and B₁ field distributions, coil sensitivity maps, motion and flow sensitivity maps, and potentially sensor data to adapt MR imaging parameters dynamically for the identified anatomic structure of region of interest, can be effectively combined with external control (Figure 3) to further enhance the performance, flexibility, and integration of the MR imaging system. The term external control refers to the ability to influence, monitor, or interact with the MR imaging system or its components from sources or systems external to the MR scanner itself. This control is typically implemented through interfaces, protocols, or auxiliary systems that extend the capabilities of the MR imaging environment to improve functionality, enhance workflow, or enable advanced applications. External control can operate at various levels, including hardware, software, or system integration. In hybrid imaging systems or MR imaging-guided interventions, external control is essential for coordinating different modalities or therapeutic systems. The optimization scheme of the invention ensures that the sequence/protocol parameters are tailored to the patient's anatomy and motion characteristics, while external control aligns these parameters with those of other systems, such as PET, CT, or focused ultrasound (HIFU). For example, motion sensitivity data can be shared with the external control system of a therapeutic device to ensure precise targeting during MR imaging-guided procedures. Moreover, external control systems often include high-performance computational platforms for real-time image reconstruction. The optimization scheme can pass B₀ and B₁ maps, along with motion and flow sensitivity distributions, to an external reconstruction algorithm, which dynamically adapts settings such as spatial resolution, noise suppression, or artifact correction thresholds. This collaboration between systems ensures that reconstructed images reflect the most accurate representation of the anatomy. Combining the optimization scheme of the invention with external control automates complex adjustments, streamlining workflows and reducing operator burden.

In a cardiac MR imaging scan, for example, the patient's heart is identified as anatomic structure of interest, an external ECG sensor provides real-time cardiac cycle data. The method of the invention uses this input along with motion sensitivity maps to predict phases of minimal motion and adjusts the protocol accordingly. Simultaneously, the method dynamically adapts the shim coils based on B₀ maps to ensure uniformity around the heart and adjust RF transmit coils based on B₁ maps to maintain consistent signal intensity. If residual motion artifacts are present, external reconstruction algorithms incorporate the motion sensitivity data to correct them in real time.

The method of the invention enhances the diagnostic utility of MR imaging by integrating advanced AI-based automation with operator-guided customization, improving workflow efficiency, image quality, and patient-specific imaging precision. The flow chart of Figure 2 in combination with Figure 3 schematically illustrates the sequential steps and decision points in one possible embodiment, emphasizing the iterative interaction between the operator, the system, and the digital twin simulation. For a more detailed explanation of the individual method steps shown in Figure 2 and optional further method steps, reference is made to the description above.

As a summary, the invention provides an intuitive AI-based selection of anatomical features allowing optimization of imaging of clinically relevant anatomical regions. A (pre-) scan that allows extraction of a B₀, a B₁ map, motion and/or flow sensitivity map can be used to optimize system and sequence adjustments and provide an essential input to reconstruction to compute an artifact-free image. Optimal planning of the region of interest, adjusting relevant coil elements and semi-automatic adjustments of the MR imaging sequences minimizing user input and optimizing clinical outcomes dependent on system (B₀, B₁), imaging sequences (flow, motion sensitivity detection, suppression) and reconstruction algorithms improve the quality of the final image. In addition, a digital twin and AI-based interactive operator manipulation of the digital twin within a user interface and the possibility to save settings allow user-intuitive exploitation of the acquired clinical MR imaging data based on the respective clinical question.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

## Claims

1. Method of magnetic resonance, MR, imaging comprising the following steps:
a) acquiring MR imaging data from a patient's anatomy by executing at least one pre-scan or scan to generate an image of the anatomy and to extract information including at least one of coil sensitivity distribution, B₀ field distribution, B₁ field distribution, spatial distribution of flow characteristics, spatial distribution of motion characteristics;
b) identifying an anatomic structure or region of interest within the imaged anatomy;
c) automatically adapting settings of an MR scanner used and/or parameters of an MR imaging sequence or protocol taking the extracted information into account for enhancing image quality or diagnostic utility for the identified anatomic structure or region of interest; and
d) acquiring further MR imaging data from the patient's anatomy using the MR imaging sequence or protocol with the adapted settings and/or parameters to generate one or more optimized images of the identified anatomic structure or region of interest.

2. The method of claim 1, further comprising the step of collecting sensor data from the patient, wherein the sensor data include at least one of physiological sensor data, motion sensor data, magnetic field sensor data, ultrasound sensor data, and wherein the sensor data are used in combination with the extracted information in the step of adapting the settings and/or parameters of the MR imaging sequence or protocol, wherein the adaptation is configured to take the sensor data into account for enhancing image quality or diagnostic utility for the identified anatomic structure or region of interest.

3. The method of claim 1 or 2, further comprising the step of determining an uncertainty map indicating the expected level and spatial distribution of residual artifacts in the one or more optimized images due to B₀ or B₁ inhomogeneity, motion and/or flow, or coil sensitivities, even after the adaptation of the MR imaging sequence or protocol parameters.

4. The method of any one of claims 1 to 3, further comprising the step of adapting settings of the MR scanner used to acquire the MR imaging data taking the extracted information into account, wherein the adaptation includes adjustments to hardware components such as gradient coils, radiofrequency coils, or shimming mechanisms to improve imaging performance for the identified anatomic structure or region of interest.

5. The method of any one of claims 1 to 4, wherein the step of identifying the anatomic structure of region of interest includes:
- presenting the generated image to a user via a user interface;
- receiving, via the user interface, a selection input from an operator; and
- processing the selection input and the image of the anatomy to identify the anatomic structure or region of interest corresponding to the input selected within the image, wherein the identification is performed by automated object selection using an image segmentation algorithm or machine learning model trained to recognize anatomic structures.

6. The method of any one of claims 1 to 5, wherein the step of identifying the anatomic structure or the region of interest includes an automated selection based on pre-saved anatomical selection data according to a pre-defined clinical question.

7. The method of any one of claims 1 to 6, further comprising the step of selecting a MR imaging sequence or protocol in an automated, semi-automated or manually executed step from a list of available MR imaging sequences or protocols suited to image the identified anatomic structure or region of interest for diagnostic purposes taking the extracted information into account for the planning and the optimization of the sequence.

8. The method of any one of claims 1 to 7, further comprising the step of automatically adapting the settings of an image reconstruction algorithm used to generate the one or more optimized images, wherein the adaptation is configured to take the extracted information and/or adapted scanner settings and/or sequence/protocol parameters into account to improve at least one of: image quality, artifact suppression, image homogeneity, image contrast, image resolution, for the identified anatomic structure or region of interest.

9. The method of any one of claims 1 to 8, further comprising the step of identifying anatomic structures or regions not relevant for clinical diagnosis, wherein the identification is used to exclude or deprioritize those anatomic structures or regions during subsequent setting of the MR scanner hardware, imaging or processing steps.

10. The method of claim 9, wherein the pre-scan or scan employs a multi-echo Dixon technique to:
- extract the B₀ field distribution within the selected anatomic structure or region of interest as well as in the non-relevant region,
- extract the B₁ field distribution within the selected anatomic structure or region of interest as well as in the non-relevant region,
- extract the coil sensitivity distribution within the selected anatomic structure or region of interest as well as in the non-relevant region, and/or
- determine the spatial distribution of motion and/or flow sensitivity within the selected anatomic structure or region of interest as well as in the non-relevant region.

11. The method of claim 10, wherein the extraction of the B₀ and/or B₁ field distribution and/or the determination of the spatial distribution of motion and/or flow sensitivity and/or coil sensitivity is performed using artificial intelligence, comprising a machine learning method trained to analyze the MR imaging data of the multi-echo Dixon pre-scan pr scan and/or the sensor data.

12. The method of any one of claims 1 to 11, further comprising the steps of:
- creating a digital twin of the patient's anatomy based on the acquired MR imaging data and the identified anatomic structure or region of interest;
- applying the adapted parameters of the MR imaging sequence or protocol, including sequence adjustments, protocol adjustments, and/or scanner settings, to the digital twin by simulating the effects of the adapted parameters on the digital twin to generate a visualization of expected image quality improvements or diagnostic enhancements; and
- presenting the visualization of the expected optimizations to the operator via the user interface, wherein the visualization allows the operator to evaluate the applied adaptations prior to execution of the MR imaging sequence or imaging protocol.

13. The method of claim 12, further comprising the steps of:
- providing the operator with a control interface to interactively modify the adapted parameters of the MR imaging sequence, protocol, or scanner settings;
- applying the operator-modified parameters to the digital twin of the patient's anatomy by simulating the effects of the operator-modified parameters on the digital twin to generate an updated visualization of the expected image quality or diagnostic outcomes; and
- presenting the updated visualization to the operator via the user interface, thereby allowing the operator to evaluate and refine the adjustments interactively prior to execution of the MR imaging sequence or protocol.

14. Magnetic resonance, MR, imaging system comprising:
- an MR scanner configured to acquire MR imaging data of a patient's anatomy;
- a processing unit operatively connected to the MR scanner and configured to:
execute at least one pre-scan or scan to generate an image of the anatomy and to extract information from the acquired MR imaging data including at least one of coil sensitivity distribution, B₀ field distribution, B₁ field distribution, spatial distribution of flow characteristics, spatial distribution of motion characteristics;
identify an anatomic structure or region of interest within the imaged anatomy; and adapt settings of the MR scanner and/or parameters of an MR imaging sequence or protocol taking the extracted information into account for enhancing image quality or diagnostic utility for the identified anatomic structure or region of interest,
wherein the MR scanner is further configured to execute the MR imaging sequence or protocol using the adapted settings and/or parameters to acquire further MR imaging data of the identified anatomic structure or region of interest, and
wherein the processing unit or a separate reconstruction unit is configured to generate an optimized MR image from the further MR imaging data.

15. Computer program comprising instructions which, when executed on a computer, cause the computer to control a magnetic resonance, MR, imaging system and to perform a method comprising:
generating an image of an anatomy and extracting information from MR imaging data acquired by at least one pre-scan or scan, the information including at least one of coil sensitivity distribution, B₀ field distribution, B₁ field distribution, spatial distribution of flow characteristics, spatial distribution of motion characteristics;
identifying an anatomic structure or region of interest within the imaged anatomy; and
adapting settings of the MR imaging system and/or parameters of an MR imaging sequence or protocol taking the extracted information into account for enhancing image quality or diagnostic utility for the identified anatomic structure or region of interest.
